# EUROPÄISCHE PATENTSCHRIFT

(11) **EP 1 514 520 B1**
(45) Veröffentlichungstag und Bekanntmachung des Hinweises auf die Patenterteilung: **19.03.2008**
(21) Anmeldenummer: 04019296.5
(22) Anmeldetag: 13.08.2004
(51) Int. Cl.: A61B 17/22, A61B 6/12

(54) **Lithotripsie-Einrichtung**
Lithotripsy device
Dispositif de lithotripsie

(30) Priorität: 11.09.2003 DE 10342016
(43) Veröffentlichungstag der Anmeldung: 16.03.2005
(73) Patentinhaber: SIEMENS AKTIENGESELLSCHAFT, 80333 München (DE)
(72) Erfinder: Saffer, Edmund, 91330 Eggolsheim (DE); Schweiger, Hans-Jürgen, 91058 Erlangen (DE)

(56) Entgegenhaltungen:
- DE-A- 10 032 982
- DE-A- 10 231 071
- DE-A- 19 526 055
- DE-C- 4 100 653
- DE-C- 4 106 370

## Beschreibung

Die Erfindung bezieht sich auf eine Lithotripsie-Einrichtung mit einer Stoßwellenquelle zum Erzeugen einer in einem Fokus fokussierten Stoßwelle und mit einer Röntgenortungseinrichtung zum Orten eines zu zerstörenden Konkrements.

Bei der Lithotripsie handelt es sich um eine therapeutische Methode, ein im Körper eines Lebewesens befindliches Konkrement ohne operativen Eingriff mit Hilfe einer fokussierten Stoßwelle zu zerstören. Um Schäden in dem das Konkrement umgebenden Gewebe weitgehend zu vermeiden, ist es einerseits erforderlich, den Fokus exakt im Konkrement zu positionieren. Andererseits ist es auch notwendig, den Fortschritt bei der Zerstörung des Konkrement zu visualisieren um die Anzahl der erforderlichen Stoßwellen-Impulse auf das therapeutisch notwenige Minimum zu begrenzen.

Zur Ortung und zur Visualisierung des Konkrements ist es beispielsweise aus der DE 197 46 956 A1 bekannt, den Lithotripter mit einer Röntgenortungseinrichtung zu koppeln. Bei dieser Röntgenortungseinrichtung handelt es sich um einen sogenannten C-Bogen, der an einem seiner Enden mit einer Röntgenquelle und am anderen Ende mit einem Röntgenempfänger versehen ist. Eine Stoßwellenquelle ist am Ende eines Tragarmes angeordnet, der passgenau und definiert mit einem an der Röntgenortungseinrichtung befindlichen Halteteil gekoppelt ist. Durch diese passgenaue Kopplung kann erreicht werden, dass der Fokus der von der Stoßwellenquelle erzeugten Stoßwelle mit dem Isozentrum des C-Bogens zusammenfällt. Der C-Bogen kann um dieses Isozentrum geschwenkt werden, so dass das Konkrement aus unterschiedlichen Projektionsrichtungen erfasst werden kann. Dies ermöglicht dessen exakte Positionierung im Isozentrum und somit im Fokus der Stoßwelle. Darüber hinaus kann auch das Fortschreiten der Zerstörung des Konkrements aus verschiedenen Richtungen überwacht werden.

Durch die statische Kopplung von Stoßwellenquelle und Röntgenortungseinrichtung ist jedoch die Richtung, mit der die Stoßwelle in den Körper des Patienten, der in der Regel auf einer höhenverstellbaren und waagrecht verschiebbaren Liege liegt, festgelegt. Abhängig von Lage und Form des Konkrements kann es jedoch zweckmäßig sein, wenn der behandelnde Arzt auch über die Möglichkeit verfügt, die Einschallrichtung flexibel zu wählen, um einen maximalen therapeutischen Erfolg bei minimaler Belastung des Patienten zu erzielen.

Aus der DE 41 06 370 C1 ist eine Lithotripsie-Einrichtung mit einem C-Bogen-Röntgengerät bekannt, die eine optische Justiereinrichtung mit an der Stoßwellenquelle angeordneten Strahlteilern und Röntgenmarkern umfasst, mit deren Hilfe eine genaue Positionierung des Konkrements im Fokus möglich ist.

Der Erfindung liegt nun die Aufgabe zugrunde, eine Lithotripsie-Einrichtung mit einer Stoßwellenquelle zum Erzeugen einer in einem Fokus fokussierten Stoßwelle und mit einer Röntgenortungseinrichtung zum Orten eines zu zerstörenden Konkrements anzugeben, mit der eine größere Flexibilität hinsichtlich der Einschallrichtung der fokussierten Stoßwelle möglich ist.

Die genannte Aufgabe wird gemäß der Erfindung gelöst mit einer Lithotripsie-Einrichtung mit den Merkmalen des Patentanspruches 1. Da die Stoßwellenquelle und die Röntgenortungseinrichtung mechanisch entkoppelt sind und eine optische Justiereinrichtung zum Überprüfen der korrekten Positionierung des Fokus auf einen relativ zur Röntgenortungseinrichtung ortsfesten Arbeitspunkt vorgesehen ist, ist es möglich, die Stoßwelle aus unterschiedlichen Richtungen in den Körper des Patienten einzukoppeln und zugleich sicherzustellen, dass der Fokus mit dem ortsfesten Arbeitspunkt zusammenfällt.

Die Röntgenortungseinrichtung umfasst eine Röntgenquelle und einen Röntgenempfänger, die gemeinsam um eine die Mittenachse der Röntgenstrahlung im Arbeitspunkt, insbesondere das Isozentrum einer als C-Bogen gestalteten Röntgenortungseinrichtung, senkrecht schneidende Schwenkachse schwenkbar sind. Dies ermöglicht eine genaue Positionierung des Konkrements im Arbeitspunkt.

Die optische Justiereinrichtung benutzt zumindest einen ersten Lichtstrahl und einen sich unter einem Winkel dazu ausbreitenden zweiten Lichtstrahl, wobei zum Erzeugen des ersten und zweiten Lichtstrahls zumindest eine Lichtquelle vorgesehen ist, die um einen Kreisbogen um den Arbeitspunkt verschiebbar an der Röntgenortungseinrichtung angeordnet ist. Dies ermöglicht eine besonders einfache variable Justierung der Stoßwellenquelle in Positionen mit unterschiedlichen Einschallrichtungen.

Die Anforderungen an die optische Justiereinrichtung und der Justieraufwand hängt wesentlich von der Anzahl der Freiheitsgrade ab, mit denen eine Positionierung (Ort und Ausrichtung) der Stoßwellenquelle möglich ist. In einer weiteren vorteilhaften Ausgestaltung ist deshalb die Stoßwellenquelle in der von der Röntgenquelle und dem Röntgenempfänger festgelegten Schwenkebene in Arbeitsstellung derart eingeschränkt positionierbar, dass ihre Mittenachse stets in der Schwenkebene liegt. Durch diese Maßnahmen sind zwar die Freiheitsgrade für die Positionierung eingeschränkt, der apparative und operative Aufwand für die Justierung ist jedoch entsprechend verringert.

Weitere vorteilhafte Ausgestaltungen der Erfindung sind in den Unteransprüchen angegeben.

Zur weiteren Erfindung wird auf das Ausführungsbeispiel der Zeichnung verwiesen. Es zeigen:
- Fig. 1 und 2: eine Draufsicht von der Seite bzw. von vorne auf eine erfindungsgemäßen Lithotripsie-Einrichtung,
- Fig. 3 bis 5: alternative Ausgestaltungen der optischen Justiereinrichtung jeweils in einer Detailansicht.

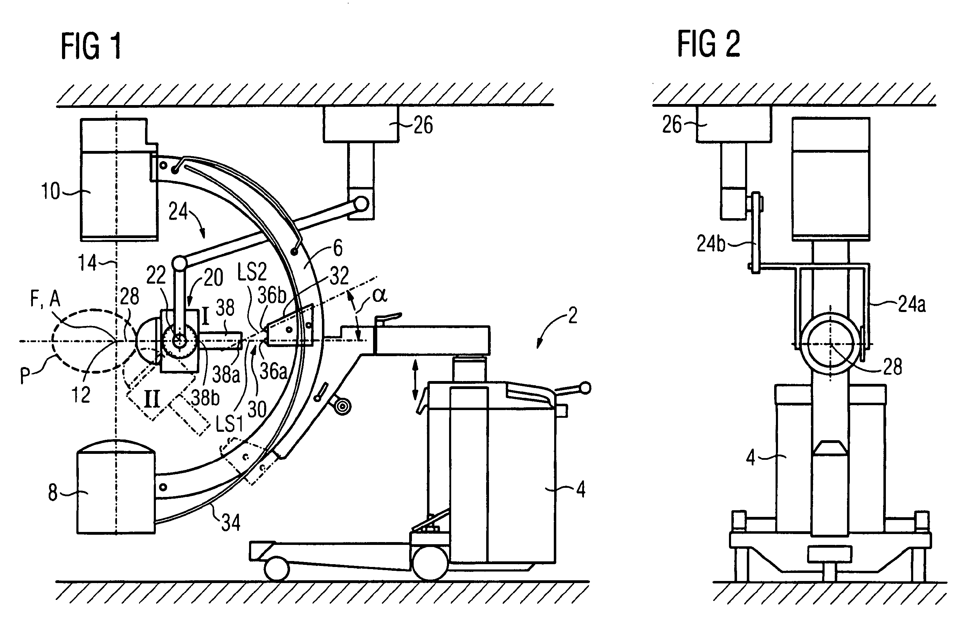

Gemäß Fig. 1 umfasst die Lithotripsie-Einrichtung eine fahrbare Röntgenortungseinrichtung 2 mit einem auf einem Gerätewagen 4 höhenverstellbar gelagerten C-Bogen 6. An einem Ende des C-Bogens 6 ist eine Röntgenquelle 8 angeordnet. Das andere Ende des C-Bogens 6 trägt einen Röntgenempfänger 10. Der C-Bogen 6 ist am Gerätewagen 4 schwenkbar um eine zur Zeichenebene senkrechte Achse 12 angeordnet, die die Mittenachse 14 der von der Röntgenquelle 8 emittierten Röntgenstrahlen in einem relativ zur Röntgenortungseinrichtung 2 ortsfesten Arbeitspunkt A schneidet, der im Ausführungsbeispiel mit dem sogenannten Isozentrum zusammenfällt. Die Röntgenquelle 8 kann somit gemeinsam mit dem Röntgenempfänger 10 um den Arbeitspunkt A geschwenkt werden, so dass durch Betrachtung aus unterschiedlichen Projektionsrichtungen festgestellt werden kann, ob sich das Konkrement im Arbeitspunkt befindet.

Im C-Bogen 6 ist eine mechanisch von diesem entkoppelte Stoßwellenquelle 20 schwenkbar um eine im Ausführungsbeispiel der Zeichnung zur Zeichenebene senkrechte Schwenkachse 22 am Ende eines Gelenkarmes 24 angeordnet, der ebenfalls schwenkbar an einer ortsfesten Trageeinrichtung 26 gelagert ist. Die Achsen des Gelenkarmes 24 und die Schwenkachse 22 sind zueinander und bei korrekter Positionierung des C-Bogens 6 relativ zur Stoßwellenquelle 20 zu dessen Schwenkachse 12 parallel und die zur Schallachse parallele Mittenachse 28 der Stoßwellenquelle 20 befindet sich in der vom C-Bogen 6 aufgespannten Ebene.

Mit Hilfe des Gelenkarmes 24 kann der Ort der Stoßwellenquelle 20 in der Schwenkebene eingestellt werden. Grundsätzlich ist es jedoch auch möglich, die Stoßwellenquelle 20 außerhalb dieser Ebene anzuordnen. Wesentlich ist jedoch, dass sie auf den Arbeitspunkt A ausgerichtet werden kann.

Die Stoßwellenquelle 20 erzeugt eine Stoßwelle, die in einem Fokus F fokussiert ist, der bei korrekter Positionierung der Stoßwellenquelle 20 mit dem Arbeitspunkt A zusammenfällt.

In der Figur sind zwei unterschiedliche Positionen I und II der Stoßwellenquelle 20 veranschaulicht, die eine Einschallung der Stoßwelle in den Körper P eines Patienten aus unterschiedlichen Richtungen bei identischer Lage des Fokus F ermöglichen. Zur korrekten Positionierung der Stoßwellenquelle 20 sind zwei Bedingungen zu erfüllen: Zum einen muss ihre Mittenachse 28 (die Mittenachse der von ihr erzeugten Stoßwelle) so ausgerichtet werden, dass diese die Mittenachse 14 der Röntgenstrahlung im Arbeitspunkt A schneidet. Zum anderen muss der Abstand der Stoßwellenquelle 20 vom Arbeitspunkt A derart eingestellt sein, dass der Fokus F mit dem Arbeitspunkt A zusammenfällt.

Um eine korrekte Positionierung zu ermöglichen, ist eine optische Justiereinrichtung 30 vorgesehen, die eine Lichtquellenanordnung 32 umfasst, die verschiebbar in einer am C-Bogen 6 angeordneten Führungsschiene 34 gelagert ist. In dieser Führungsschiene 34 kann die Lichtquellenanordnung 32 in einer Kreisbahn um die Schwenkachse 12 gedreht werden und in frei wählbaren Positionen arretiert werden. Alternativ hierzu ist es auch möglich, am C-Bogen 6 eine Mehrzahl von Rastpositionen für die Lichtquellenanordnung 32 vorzusehen.

Die Lichtquellenanordnung 32 umfasst eine erste Lichtquelle 36a und eine zweite Lichtquelle 36b, die einen ersten bzw. zweiten Lichtstrahl LS1 bzw. LS2 erzeugen, die sich schiefwinklig unter einem Winkel α zueinander ausbreiten. Der erste Lichtstrahl LS1 breitet sich in der von der Kreisbahn aufgespannten Ebene radial zum Kreismittelpunkt und senkrecht zur Schwenkachse 12 aus, so dass er diese in einem Schnittpunkt außerhalb der Schwenkebene schneidet. Erste und zweite Lichtquelle 36a,b sind vorzugsweise Laserstrahlquellen, die einen Laserstrahl erzeugen, der am Auftreffpunkt einen scharf begrenzten, gut erkennbaren Lichtfleck erzeugt. Erste und zweite Lichtquelle 36a,b sind ortsfest zueinander in der Lichtquellenanordnung 32 angeordnet und können nur gemeinsam in der Führungsschiene 34 verschoben werden.

Zur korrekten Ausrichtung der Stoßwellenquelle 20 auf den Arbeitspunkt A ist diese mit einer Visiereinrichtung 38 versehen, die zwei einander gegenüberliegende Justiermarken 38a,b umfasst. Im Ausführungsbeispiel ist die Visiereinrichtung 38 ein für den ersten Lichtstrahl LS1 transparenter Körper der an seinen gegenüberliegenden Stirnseiten mit Markierungen versehen ist. Die Justiermarken 38a,b legen eine Gerade fest, die parallel zur Mittenachse 28 der Stoßwellenquelle 20 verläuft. Der Abstand dieser Geraden zur Mittenachse 28 entspricht dabei dem Abstand der von der Führungsschiene 34 festgelegten Ebene, in der sich die Lichtstrahlen LS1,LS2 ausbreiten, vom Arbeitspunkt A.

Anstelle einer aus einem massiven transparenten Körper aufgebauten Visiereinrichtung 38 kann diese auch aus gegenüberliegenden Lochblenden oder aus einer Lochblende und einer gegenüberliegenden Markierung aufgebaut sein.

Trifft der erste Lichtstrahl LS1 auf beide Justiermarken 38a,b, so ist die Stoßwellenquelle 20 korrekt auf den Arbeitspunkt A ausgerichtet.

Der korrekte Abstand der Stoßwellenquelle 20 vom Arbeitspunkt A ist dann eingestellt, wenn der zweite Lichtstrahl LS2 ebenfalls auf die erste Justiermarke 38a trifft.

Anstelle der in der Figur dargestellten Anordnung ist es auch möglich, die dem zweiten Lichtstrahl zugeordnete Justiermarke an einer anderen Stelle an der Stoßwellenquelle 20 zu positionieren.

Im Ausführungsbeispiel sind erster und zweiter Lichtstrahl LS1, LS2 sowie die zugehörigen Justiermarken 38a,b derart angeordnet, dass die Mittenachse 28 der Stoßwellenquelle 20 bei korrekter Positionierung in der Schwenkebene des C-Bogens 6 liegt. Grundsätzlich ist aber auch jede andere Ebene realisierbar.

Im Ausführungsbeispiel stehen zur Positionierung der Stoßwellenquelle 4 Freiheitsgrade zur Verfügung (freie Positionierbarkeit in x-, y- und z- Richtung und freie Rotation um die Schwenkachse 22), von der nach korrekter Einstellung der Grundposition von Stoßwellenquelle 20 und Röntgenortungseinrichtung 2 (Mittenachse der Stoßwellenquelle 20 befindet sich in der Schwenkebene) einer, nämlich der translatorische Freiheitsgrad senkrecht zur Zeichenebene, festliegt. Es sind jedoch auch Anordnungen vorstellbar, die eine größere Anzahl von Freiheitsgraden für die Positionierung der Stoßwellenquelle 20 ermöglichen, beispielsweise durch deren kardanische Lagerung am Ende des Gelenkarmes 24. Wesentlich ist jedoch, dass die Anzahl der Freiheitsgrade bei der Positionierung der Stoßwellenquelle 20 und die Anzahl und Anordnung der Justiermarken 38a,b sowie die Ausbreitungsrichtungen und Anzahl der zur Justierung verwendeten Lichtstrahlen LS1, LS2 derart aufeinander abgestimmt sind, dass bei korrekter Positionierung aller Justiermarken sichergestellt ist, dass der Fokus F der Stoßwellenquelle 20 mit dem Arbeitspunkt A zusammenfällt.

In der Draufsicht gemäß Fig. 2 ist deutlich zu erkennen, dass die Mittenachse 28 der Stoßwellenquelle 20 in der senkrecht zur Zeichenebene verlaufenden Schwenkebene verläuft. Um eine solche Positionierung problemlos zu ermöglichen, ist der die Stoßwellenquelle 20 tragende Unterarm 24a des Gelenkarmes 24 seitlich versetzt zum Oberarm 24b an diesem angelenkt.

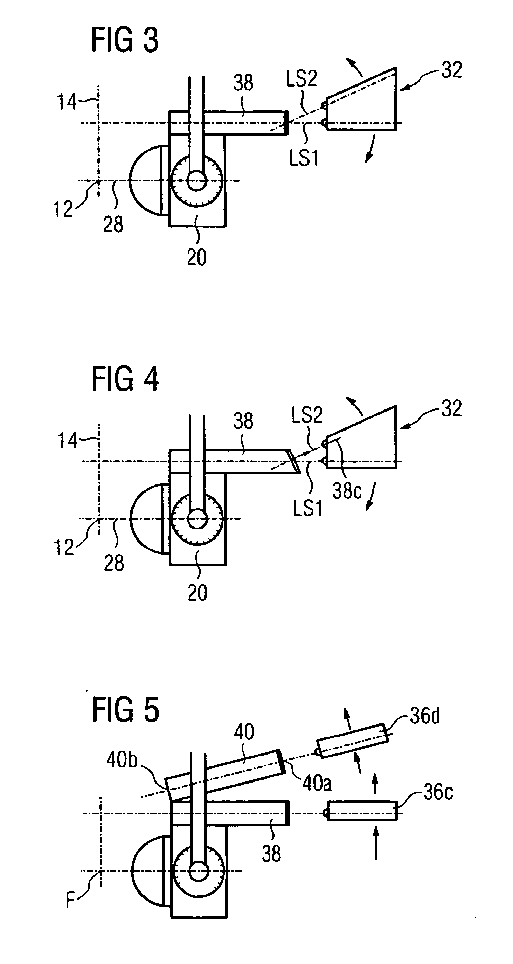

Im Ausführungsbeispiel gemäß Fig. 3 ist eine alternative Anordnung der Visiereinrichtung 38 dargestellt, bei der diese oberhalb der Stoßwellenquelle 20 angeordnet ist, so dass der von ihr emittierte erste Lichtstrahl LS1 bei korrekter Positionierung außerhalb des Arbeitspunktes A nicht die Schwenkachse 12 des C-Bogens 6 sondern die Mittenachse 14 der Röntgenstrahlen schneidet.

In der alternativen Ausgestaltung gem. Fig. 4 umfasst die Lichtquellenanordnung 32 lediglich eine einzige Lichtquelle 36, die einen ersten Laserstrahl LS1 emittiert. Die Visiereinrichtung 38 ist in diesem Fall aus einem prismatischen Körper aufgebaut, dessen der Lichtquellenanordnung 32 zugewandten Stirnfläche 39 schräg zur Ausbreitungsrichtung des ersten Lichtstahls LS1 orientiert ist. Ein Teil des ersten Lichtstrahl LS1 wird an der Lichtquellenanordnung 32 zugewandten Stirnfläche reflektiert und gelangt im Ausführungsbeispiel zur Lichtquellenanordnung 32 zurück. Diese ist mit einer Justiermarke 38c versehen. Trifft der reflektierte Lichtstrahl, der dem zweiten Lichtstrahl LS2 der vorherigen Ausführungsbeispiel entspricht, auf diese Justiermarke 38c, so ist die korrekte Abstandsposition ebenso wie in den vorstehenden Ausführungsbeispielen erreicht.

Anstelle der in den Ausführungsbeispielen gemäß Fig. 1 bis 4 gezeigten Verwendung zweier ortsfest zueinander angeordneter Lichtquellen, ist auch die Verwendung zweier unabhängig voneinander auf der Führungsschiene verfahrbaren Lichtquellen 36c,d möglich. Hierzu ist es beispielsweise lediglich erforderlich an der Stoßwellenquelle 20 eine zweite baugleiche Visiereinrichtung 40 mit entsprechenden Justiermarken 40a,b anzuordnen, die unter einem vorgegebenen Winkel zur Visiereinrichtung 38 angeordnet ist, wie dies in Fig. 5 veranschaulicht ist. Auch in dieser Anordnung kann ebenso wie in den Ausführungsbeispielen der Fig. 1 bis 4 die Ausrichung der Stoßwellenquelle 20 auf den Fokus unabhängig von der Einstellung ihres Abstandes vom Fokus F vorgenommen werden.

Grundsätzlich ist es auch möglich, Lichtquellenanordnungen 32 und Visiereinrichtung zu vertauschen.

## Patentansprüche

1. Lithotripsie-Einrichtung mit einer Stoßwellenquelle (20) zum Erzeugen einer in einem Fokus (F) fokussierten Stoßwelle und mit einer davon mechanisch entkoppelten Röntgenortungseinrichtung (2) zum Orten eines zu zerstörenden Konkrements, die eine Röntgenquelle (8) und einen Röntgenempfänger (10) umfasst, die gemeinsam um eine die Mittenachse (14) der Röntgenstrahlung in einem relativ zur Röntgenortungseinrichtung (2) ortsfesten Arbeitspunkt (A) senkrecht schneidende Schwenkachse (12) schwenkbar sind, sowie mit einer optischen Justiereinrichtung (30) zum Überprüfen der korrekten Positionierung des Fokus (F) auf den Arbeitspunkt (A), wobei die optische Justiereinrichtung (30) zumindest eine Lichtquelle zum Erzeugen zumindest eines ersten Lichtstrahls (LS1) und eines sich unter einem Winkel (α) dazu ausbreitenden zweiten Lichtstrahls (LS1), denen jeweils Justiermarken (38a,b; 38c) zugeordnet sind, umfasst, **dadurch gekennzeichnet, dass** die zumindest eine Lichtquelle in einer Kreisbahn um die Schwenkachse (12) schwenkbar an der Röntgenortungseinrichtung (2) angeordnet ist.

2. Lithotripsie-Einrichtung nach Anspruch 1, bei der die Stoßwellenquelle (20) in der von der Röntgenquelle (8) und dem Röntgenempfänger (10) festgelegten Schwenkebene in Arbeitsstellung derart eingeschränkt positionierbar ist, dass ihre Mittenachse (28) stets in der Schwenkebene liegt.

3. Lithotripsie-Einrichtung nach Anspruch 1 oder 2, bei der erster und zweiter Lichtstrahl (LS1, LS2) relativ zueinander ortsfest sind.

4. Lithotripsie-Einrichtung nach Anspruch 1, 2 oder 3, bei der die Stoßwellenquelle (20) mit Justiermarken (38a,b) für den ersten und zweiten Lichtstrahl (LS1, LS2) versehen ist.

5. Lithotripsie-Einrichtung nach einem der Ansprüche 1 bis 4, bei der die optische Justiereinrichtung (30) eine aus einem Prismenkörper bestehende Visiereinrichtung (38) umfasst, der den auf ihn auftreffenden ersten Lichtstrahl (LS1) in einen transmittierten Lichtstrahl und einen reflektierten Lichtstrahl aufteilt, wobei dieser den zweiten Lichtstrahl (LS2) bildet, und bei der die Justiermarke (38c) für den zweiten Lichtstrahl (LS2) ortsfest relativ zur Lichtquellenanordnung (32) angeordnet ist.

## Claims

1. Lithotripsy device having a shockwave source (20) for generating a shockwave focused at a focus (F) and having an X-ray localization device (2) mechanically decoupled therefrom for localizing a concrement to be disintegrated, which comprises an X-ray source (8) and an X-ray receiver (10) that can be swivelled together about a swivel axis (12) perpendicularly intersecting the mid-axis (14) of the X-radiation at a point (A) which is stationary relative to the X-ray localization device (2), and having an optical alignment device (30) to verify correct positioning of the focus (F) on the working point (A), wherein the optical alignment device (30) comprises at least one light source for generating at least a first light beam (LS1) and a second light beam (LS2) propagating at an angle (α) relative thereto, to which alignment marks (38a,b; 38c) are respectively assigned, **characterized in that** the at least one light source is arranged so that it can swivel in a circular path about the swivel axis (12) on the X-ray localization device (2).

2. Lithotripsy device according to Claim 1, wherein the shockwave source (20) can be positioned in a restricted way in the swivel plane established by the X-ray source (8) and the X-ray receiver (10) in an operational setting, such that its mid-axis (28) always lies in the swivel plane.

3. Lithotripsy device according to Claim 1 or 2, wherein the first and second light beams (LS1, LS2) are stationary relative to one another.

4. Lithotripsy device according to Claim 1, 2 or 3, wherein the shockwave source (20) is provided with alignment marks (38a,b) for the first and second light beams (LS1, LS2).

5. Lithotripsy device according to one of Claims 1 to 4, wherein the optical alignment device (30) comprises a sighting device (38) consisting of a prism body, which splits the first light beam (LS1) incident on it into a transmitted light beam and a reflected light beam, the latter forming the second light beam (LS2), and wherein the alignment mark (38c) for the second light beam (LS2) is arranged stationary relative to the light source arrangement (32).

## Revendications

1. Dispositif de lithotripsie, comprenant une source (20) d'onde de choc pour la production d'une onde de choc focalisée dans un foyer (F) et un dispositif (2) de localisation par rayons X, qui en est découpée mécaniquement, qui est destiné à localiser un concrément à détruire et qui comprend une source (8) de rayons X et un récepteur (10) de rayons X pouvant pivoter conjointement autour d'un axe (12) de pivotement coupant perpendiculairement l'axe (14) médian du rayonnement x en un point (A) de travail fixe en position par rapport au dispositif (2) de localisation par rayons X, ainsi qu'un dispositif optique de réglage pour contrôler le positionnement correct du foyer (F) sur le point (A) de travail, dans lequel le dispositif (30) optique de réglage comprend au moins une source lumineuse de production d'un premier faisceau (LS1) lumineux et d'un deuxième faisceau (LS1) lumineux s'en écartant d'un angle (α) et auxquels sont associés respectivement des repères (38 a, b, 38 c) de réglage, **caractérisé en ce que** la au moins une source lumineuse est montée pivotante sur le dispositif (2) de localisation par rayons X dans une trajectoire autour de l'axe (2) de pivotement.

2. Dispositif de lithotripsie suivant la revendication 1, dans lequel la source (20) d'onde de choc peut être mise en position, de manière limitée, dans le plan de pivotement fixé par la source (8) de rayons X et par le récepteur (10) de rayons X, en position de travail de façon à ce que son axe (28) médian soit toujours dans le plan de pivotement.

3. Dispositif de lithotripsie suivant la revendication 1 ou 2, dans lequel, le premier et le deuxième faisceaux (LS1, LS2) lumineux sont fixes en position l'un par rapport à l'autre.

4. Dispositif de lithotripsie suivant la revendication 1, 2 ou 3, dans lequel la source (20) d'onde de choc est munie de repères (38a, b) de réglage du premier et du deuxième faisceaux (LS1, LS2) lumineux.

5. Dispositif suivant l'une des revendications 1 à 4, dans lequel le dispositif (30) optique de réglages comprend un dispositif (38) de visée constitué d'un prisme qui divise le premier faisceau (LS1) lumineux qui arrive sur lui en un faisceau lumineux transmis et en un faisceau lumineux réfléchi, celui ci formant le deuxième faisceau (LS2) lumineux, et dans lequel le repère (38c) de réglage du deuxième faisceau (LS2) lumineux est disposé en position fixe par rapport au montage (32) ayant la source lumineuse.
